# EUROPEAN PATENT SPECIFICATION

(11) **EP 2 654 662 B1**
(45) Date of publication and mention of the grant of the patent: **22.03.2017**
(21) Application number: 11850698.9
(22) Date of filing: 20.12.2011
(51) Int. Cl.: A61J 15/00, A61M 25/095, A61M 25/092, A61M 39/08, A61B 17/34, A61B 1/00, A61B 1/005, A61M 25/01

(54) **OPTICALLY GUIDED MEDICAL TUBE AND CONTROL UNIT ASSEMBLY AND METHODS OF USE**
OPTISCH GESTEUERTER MEDIZINISCHER TUBUS UND STEUEREINHEIT SOWIE VERFAHREN ZUR VERWENDUNG
ENSEMBLE TUBE MÉDICAL GUIDÉ OPTIQUEMENT ET UNITÉ DE COMMANDE ET PROCÉDÉS D'UTILISATION

(30) Priority: 21.12.2010 US 201061425730 P
(43) Date of publication of application: 30.10.2013
(60) Divisional application: 17153483.7
(73) Proprietor: University of Utah Research Foundation, Salt Lake City, UT 84108 (US)
(72) Inventor: NIEMAN, Timothy, North Salt Lake, UT 84054 (US); PERRY, Trent, North Salt Lake, UT 84054 (US)
(74) Representative: Bennett, Nicholas
(86) International application number: PCT/US2011/066324
(87) International publication number: WO 2012/088201

(56) References cited:
- WO-A1-2009/108854
- DE-U1- 29 620 135
- US-A1- 2007 203 393
- US-A1- 2009 062 772
- US-A1- 2009 318 757
- US-A1- 2010 305 503
- US-A1- 2010 305 503

## Description

### THE FIELD OF THE INVENTION

The present invention relates to devices used to view an area when direct line-of-sight observation is not feasible. In some aspects, the invention relates to an optically guided medical tube and control unit assembly that may be used for prolonged and/or repeated viewing of an area that would otherwise be difficult or impossible to view with the naked eye, such as the interior anatomy of a human or animal. In other aspects, the disclosure relates to methods of using an optically guided medical tube and control unit assembly.

### BACKGROUND

Medical tubes are used in a variety of medical procedures that require one end of the tube to be located inside the body while the other end remains outside the body. Such tubes can be useful in treating a patient over an extended period of time by, for example, repeated administration of medication, delivery of nutrients or oxygen, or removal of fluids. Proper placement and positioning of medical tubes is often critical to their effective use. For example, it is typically desirable to administer medication or deliver nutrients to a specific location in the body to maximize the efficacy of the medication or the benefits of the tube. However, placement of most medical tubes is often performed without immediate visual confirmation that the tube has been located at the proper location.

For example, medical tubes are often used to treat a patient if the patient has a compromised ability to obtain proper nutrition through oral intake. The medical tubes used for this treatment are more commonly referred to as nasogastric or nasoenteric feeding tubes. Placement of these feeding tubes is routinely performed in a number of clinical settings including emergency rooms, hospital wards and intensive care units totaling greater than 1.2 million tubes annually. Feeding tube placement, like placement of most other medical tubes, is commonly done without a visual aid to help medical personnel navigate the tube down the nose, through the esophagus and into the stomach or small intestine and confirm that the distal end of the tube is placed in its proper end location. Medical personnel advance the tubes through the patient's body blindly. Because the medical personnel cannot see the distal end of the feeding tube during advancement, the feeding tube can be incorrectly positioned during the process. In extreme cases, the distal end of the feeding tube may pass into the cranium and into the patient's brain, while the nurse or other practitioner continues advancing the feeding tube believing that it is properly entering the gastrointestinal tract.

More commonly, misplacement of the feeding tube results in other serious complications including lung placement or puncture or esophageal puncture. It is estimated that 3.2 percent of all blind nasoenteric feeding tube placements result in the feeding tube being disposed in the lung. In approximately 1.2 percent of placements, the patient will suffer a punctured lung. In approximately 0.5 percent of cases, the patient will die as a result of the procedure. It is estimated that in intensive care units alone, up to six thousand patients die each year from improperly placed feeding tubes.

Additionally, providing any feeding solution through the feeding tube into the lungs likely results in pneumonia with increased morbidity and mortality. Thus, it is critical to ensure verify that there has been proper placement of the feeding tube. Unfortunately, many common methods for doing so leave patients at substantial risk.

Proper placement of the tube is verified using a variety of tests, including chest x-ray, pH tests, auscultation, or fluoroscopy. However, these tests only attempt to confirm position after placement when complications may have already occurred. For example, if fluoroscopy or X-ray confirms that the feeding tube is actually disposed in the lung, it does so only after the possibility of lung puncture or other damage to the lung tissue. Additionally, while X-ray imaging and fluoroscopy are often used, both only provide a two dimensional indication of location, i.e. placement below the diaphragm. In multiple instances, confirmation of placement has been given when the feeding tube had actually passed through the lung and along the diaphragm, rather than being disposed in the gastrointestinal tract. Moreover, X-ray or fluoroscopic confirmation does not clearly confirm placement in the small bowel rather than the stomach. Small bowel placement is generally preferred to prevent the risk of aspirating feeding solution.

Additionally, some of these techniques have additional limitations and drawbacks. For example, fluoroscopic exams and X-Ray verification can cost $400 or more and can expose the patient and the practitioner to harmful radiation. If a patient is pregnant, a child or in poor health, exposure to such radiation may be highly undesirable. Additionally, the use of such verification procedures significantly prolongs the period of time that a patient must wait after a feeding tube is placed before feeding can begin. Because of this, the average time from ordering feeding tube placement to confirmation of placement and beginning of feeding is 22-26 hours. If the tube is placed improperly, the wait to begin feeding can take even longer as the process must be repeated. During this time, the patient is unable to obtain nourishment and any medications which may be delivered via a feeding tube.

Another complication which is common with patients receiving a feeding tube is that the patients are often not coherent. The patient may be partially sedated or may be delirious. Thus, it is not uncommon for a patient to pull out a feeding tube which has previously been placed. This requires repetition of the procedure, again subjecting the patients to the risks set forth above. Thus, a simpler, safer method for placing feeding tubes would be highly desirable.

An alternate method for placement and verification of feeding tubes is by use of an endoscope. Typically an endoscope is inserted into the mouth of the patient and advanced down until the endoscope has passed through the esophagus and at least into the stomach, and preferably through the pyloric sphincter and into the duodenum. In some applications, a guidewire is advanced to the proper location and the endoscope is removed. The guidewire is then manipulated to move it from the oral placement to a nasal placement, and a feeding tube is advanced along the guidewire into the desired location.

In other applications, the feeding tube is carried in a working channel (or along the side) of the endoscope. The feeding tube is sufficiently long that once the feeding tube has been placed, the endoscope can be removed over the feeding tube. The feeding tube is then cut and an appropriate adaptor attached for feeding.

While placement and verification using an endoscope is advantageous, it also has several drawbacks. One drawback is that using an endoscope usually takes considerable skill to steer the tip of the endoscope through complex and tortuous paths in the body, which can be made more difficult if the tip cannot be steered in multiple directions. Furthermore, endoscopy procedures are typically performed by physicians, often requiring a longer wait time before a properly trained physician is available to place the feeding tube, as opposed to the wait time if medical personnel other than a physician could place the tube.

Another drawback of using an endoscope to place a feeding tube is that, because the endoscope is typically placed through the mouth, an additional procedure must be used if the feeding tube is to be used nasoenterically. This involves advancing a structure through the nose and out the mouth, securing the end of the feeding tube (or a guidewire) to the structure, and then pulling the structure and the end of the feeding tube through the nose. Also, the procedure usually requires conscious sedation which increases the risk and cost of the procedure.

Additionally, an endoscope is a complex, expensive medical device. One significant cost associated with the manufacture of an endoscope can be the optical fibers used to transmit light and images. Devices that are designed to inspect the inside of the body of a human or animal are typically designed to allow medical personnel to perform additional functions inside the patient, other than just viewing the internal anatomy. Additionally, these devices must be able to navigate through narrow passageways in the body. Thus an optical fiber that is appropriate for use in such devices must be sufficiently narrow so that the device and any additional lumen for passing instruments, wires, etc., can comfortably traverse through narrow passageways in the body. Optical fiber with a small enough diameter, however, is typically more expensive. For example, the cost of an optical fiber made essentially of glass (1.0 OD) can be more than four times greater than the cost of an optical fiber made of plastic (1.8 - 2.0 OD) which performs similarly as the smaller, glass optical fiber. The larger optical diameter of the cheaper, plastic optical fiber, however, may limit the amount of space for additional lumens that may be used to perform additional functions.

Furthermore, because an endoscope is a complex and expensive medical device it is generally not discarded after use in one patient, but rather it is reused in subsequent patients. Prior to reuse of the endoscope, however, it must be properly prepared and sterilized to reduce the risk that an infection could be transmitted from one patient to another. The procedure of cleaning and disinfecting an endoscope is both time consuming and expensive and may involve mechanical cleaning, leakage testing, disinfecting the endoscope chemically for an appropriate amount of time, and then rinsing and drying the endoscope. Often this process must be accomplished by an individual properly qualified to perform the procedure.

Each of the above-referenced methods for placing a feeding tube also has problems with subsequently confirming that the feeding tube remains properly placed. As a patient moves, the distal end of the feeding tube can work its way out of the intestine and coil in the stomach. Depending on the particular concerns regarding the patient, it may be necessary to periodically confirm that the feeding tube is placed properly. This can require additional x-ray, pH tests, auscultation, or fluoroscopy, or the use of another endoscope to ensure that the feeding tube is properly placed. Each of these methods for confirming placement has the drawbacks mentioned above.

Although the current discussion has been directed at the disadvantages of current methods for placing a feeding tube, similar disadvantages exist with current procedures for placing other medical tubes. Such other procedures include placement of a jejunal extension tube in percutaneous gastrojejunal feeding tubes (PEGJ). Presently, for PEGJ tubes, the jejunal extension tube must be threaded through the existing gastrostomy tube or stoma into the small bowel. This is done using fluoroscopy or endoscopy to advance a wire into the small intestine and then a jejunal feeding tube is passed over the wire into the small intestine (jejunum). A jejunal extension tube with direct visualization and/or steering mechanism could perform the same task without the drawbacks of using endoscopy or fluoroscopy as noted previously.

Other situations include those in which prolonged visualization and access for irrigation/infusion and drainage would be beneficial. For example, in pancreatobiliary infections, it is not uncommon for the common bile duct (or associated duct) to become blocked and restrict fluid flow into the duodenum. An endoscope or other catheter based method is typically used to place a shunt or stent in the pancreatic duct or the common bile duct to allow proper drainage of pus from the pancreatobiliary ducts through the common bile duct into the duodenum. Once the situation has been alleviated, an endoscope or other device may be advanced back into the duodenum to remove the shunt or stent. Of course, it is often difficult to tell if the situation has been fully alleviated, if the device has become misplaced, or if the symptoms have simply been reduced.

In these procedures, as well as others in the body, it may be advantageous to provide continued viewing capacity both to ensure proper placement of the structure used for drainage, and to allow medical personnel to get a view of the affected area to determine whether and how quickly healing and/or drainage is taking place. In these clinical situations such a device can replace or assist fluoroscopy and/or endoscopy for guidance, placement, confirmation and reconfirmation. In addition, such an indwelling device can be used to drain pus or other bodily fluids from body cavities as well as provide a conduit for irrigation and infusion of medications including antibiotics.

Accordingly, it would be desirable to provide a medical tube which can be placed more conveniently and which can be used to confirm placement without the need for radiation or other traditional confirmation methods. Additionally, it would be advantageous if such medical tubes could be used, and methods for placing such medical tubes could be performed, by medical personnel other than physicians.

Thus, there is a need for an improved optically guided medical tube and control unit assembly and methods of using the same that reduces risks associated with the placement of medical tubes inside the body of a patient. The improved optically guided medical tube and control unit assembly should be readily reusable to confirm that the distal end of a medical tube has not been displaced after the medical tube has been associated with a patient over an extended period of time, for example about 30 minutes or more. It is desirable that the optically guided medical tube and control unit assembly is relatively easy to use and that it is relatively inexpensive to manufacture.

The document US2010/0305503 A1 discloses means for placing and verifying proper placement of nasogastric and nasoenteric feeding tubes. The document US2009/0318757 A1 discloses apparatus for intubating a patent. The apparatus comprises a pair of longitudinally extending lumens including a sensor channel and a working channel. The document DE29620135 discloses a probe for removing mucus and other fluids from the stomach.

### SUMMARY OF THE INVENTION

The invention is defined in independent apparatus claim 1 and its dependent claims 2 - 11.

### BRIEF DESCRIPTION OF THE DRAWINGS

Various embodiments of the present invention are shown and described in reference to the numbered drawings wherein:
FIG. 1 shows a fragmented perspective view of an optical tube and control unit assembly made in accordance with the principles of the present invention;
FIG. 2 shows an alternate perspective view of the optical tube and control unit assembly of FIG. 1;
FIG. 3 shows a fragmented perspective view of an optical medical tube and coupling adaptor of the present invention;
FIG. 4 shows a fragmented perspective view of another optical medical tube and coupling adaptor of the present invention;
FIG. 5 shows a fragmented perspective view of yet another optical medical tube of the present invention;
FIG. 6 shows a perspective view of the coupling adaptor of the optical medical tube in FIG. 5;
z shows an end view of a tube of an optical medical tube made according to principles of the present invention;
FIG. 7B shows an end view of another tube and stylet of an optical medical tube made according to principles of the present invention;
FIG. 8 show a close-up perspective view of an optical medical tube with a connector associated therewith being disconnected from a coupling adaptor;
FIG. 9A shows a perspective view of a tube mounting unit and a tubing segment according to one aspect of the present invention;
FIG. 9B shows an alternate perspective view of the tube mounting unit of FIG 9A having a tubing segment in a different channel;
FIG. 9C shows a close-up view of an occlusion member and retention member occluding a tubing segment as shown in FIG. 9A;
FIG. 9D shows a close-up view of an occlusion member and retention member as shown in FIG. 9B;
FIG. 10 shows a side, partially cut-away view of a control unit with its actuation mechanisms exposed formed according to principles of the present invention;
FIG. 11A shows a perspective view of another tube mounting unit according to one aspect of the present invention;
FIG. 11B shows a side view of the tube mounting unit shown in FIG. 11A with an occluded tubing segment;
FIG. 11C shows another side view of the tube mounting unit shown in FIG. 11 A;
FIG. 12 shows a side view of a tubing section made in accordance with principles of the present invention;
FIG. 13A shows a fragmented, close-up top view of a steering mechanism having a wheel coupled to a control unit of an optical tube and control unit assembly of the present invention;
FIG. 13B shows a fragmented, perspective view of a coupling adaptor for connecting the optics and steering mechanism of an optical medical tube with a control unit assembly;
FIG. 14 shows a fragmented, perspective view of a coupling adaptor being removably connected to a control unit according to principles of the present invention; and
FIG. 15 shows a perspective, cutaway view of a stylet and tube tip of a medical tube according to principles of the present invention.

It will be appreciated that the drawings are illustrative and not limiting of the scope of the invention which is defined by the appended claims. The embodiments shown accomplish various aspects and objects of the invention. It is appreciated that it is not possible to clearly show each element and aspect of the invention in a single figure, and as such, multiple figures are presented to separately illustrate the various details of the invention in greater clarity. Thus, it may be considered that any aspect shown in one drawing could be used in connection with structures shown in another drawing. Similarly, every embodiment need not accomplish all advantages of the present invention.

### DETAILED DESCRIPTION

The drawings will now be discussed in reference to the numerals provided therein so as to enable one skilled in the art to practice the invention. The drawings and descriptions are exemplary of various aspects of the invention and are not intended to narrow the scope of the appended claims.

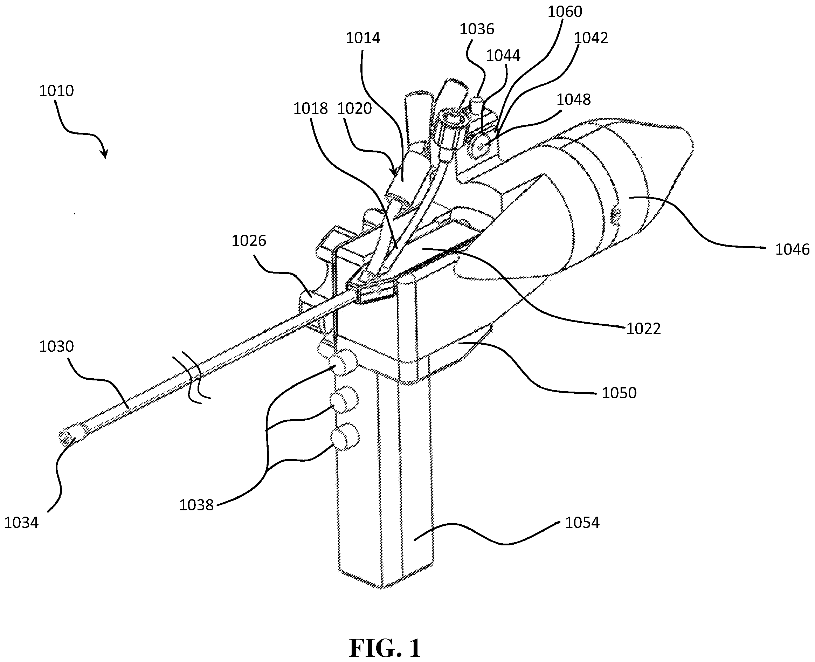

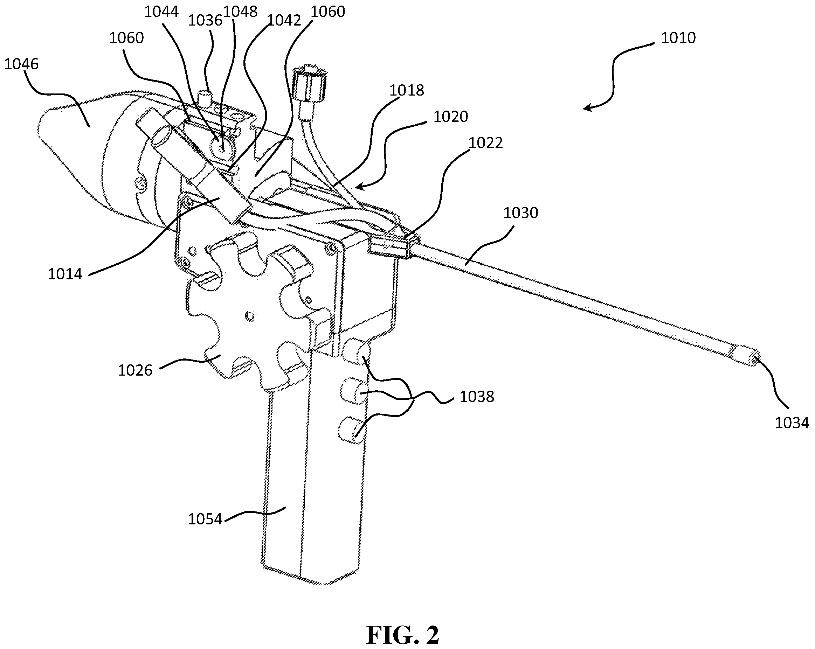

Now turning to FIGs. 1 and 2, there is shown perspective views of an optical tube and control unit assembly, generally indicated at 1010, made in accordance with the principles of the present invention. The assembly 1010 may include a medical tube 1030 comprised of an imaging device 1034 and steering members (not shown), and a control unit 1050. The medical tube 1030 may be, for example, a nasoenteric feeding tube or other tube for the delivery of nutrients or other solutions to a patient. (The tube 1030 may be discussed interchangeably herein as an optical tube or medical tube as it may be used for purposes outside of a medical context if desired.)

The control unit 1050 is removably connectable to the medical tube 1030 and may include a tube mounting unit 1042, a handle 1054, a steering control, such as a thumbwheel 1026, and a housing 1046 which may contain an image capturing device and/or light source. One advantage of one aspect of the present invention is that the control unit may be used independent of a given medical tube 1030. Thus, for example, a physician may carry a control unit and use the unit with multiple medical tubes disposed in a number of patients without the need to sterilize the control unit 1050.

When the medical tube 1030 is coupled to the control unit 1050 the imaging device 1034, such as an optical fiber, may be aligned so as to allow light from a light source located in housing 1046 to be directed through the optical fiber 1034 to a distal end of the medical tube 1030 to thereby provide light as the medical tube 1030 is advanced along a pathway. Additionally, the imaging device 1034 may be aligned so as to transmit information to the imaging capture device which may be located in housing 1046, thus allowing the user of the assembly 1010 to view an area when direct line-of-sight observation would otherwise be difficult or impossible. For example, medical personnel using the assembly 1010 would be able to visually navigate a tube 1030 down the nose, through the esophagus and into the stomach or small intestine, in order to place a feeding tube for treatment of a patient. By providing the medical personnel the ability to see as he or she advances the tube 1030, assembly 1010 may reduce the risk that the patient will be injured during the placement procedure, decrease the time necessary to place the tube, and allow for immediate confirmation that the tube 1030 is properly placed. Thus, the risk of a cranial or lung injury to a patient may be dramatically reduced and the time and expense of confirming proper placement may be reduced from hours to minutes.

The optical tube and control unit assembly 1010 may also include a steering mechanism to facilitate navigation of the tube 1030 through a tortuous pathway. The steering mechanism (which is explained in more detail below) may include the medical tube 1030 having one or more wires which are operationally connectable to the control unit and which may be used to steer the tubing 1030 using a steering control such as, for example, thumbwheel 1026, a wheel, lever, etc.

As can be seen in FIGs. 1 and 2, tube 1030 may be coupled (either permanently or temporarily) to a coupling adaptor 1022, which is configured for removably connecting to the control unit 1050. The coupling adaptor 1022 may allow quick and simple engagement of the steering mechanism with the control unit 1050 and alignment of the imaging device 1034 with the image capturing device and light source in housing 1046. As explained in further detail below, engagement of the steering mechanism and alignment of the imaging device may occur simultaneously upon placement of coupling adaptor 1022 in or on the control unit 1050, such as in a recess. Once the medical tube 1030 is guided to its proper location, coupling adaptor 1022 can be disconnected from control unit 1050, enabling the control unit to be used with other medical tubes. Because the control unit 1050 may not come into direct contact with the body, the control unit 1050 may be cleaned by simply wiping it with an antiseptic wipe rather than requiring a more complex sterilization procedure. This will likely reduce both time and expense for medical personnel and the patient.

Coupling adaptor 1022 may be constructed so as to be lightweight and small enough to go substantially unnoticed when the medical tube 1030 is intended to be associated with a patient for a prolonged period of time. Thus, the coupling adaptor 1022 may remain on the medical tube 1030 with minimal, if any, disturbance of the patient. Medical personnel may easily reconnect the coupling adaptor 1022 to the control unit 1050 at a later time in order to confirm that the medical tube 1030 has not been inadvertently displaced, to view tissue surrounding the distal end of the medical tube, to change position of the medical tube, or perform other acts or procedures as is necessary.

It will be appreciated that the ability to easily engage and disengage the coupling adaptor 1022 from the control unit 1050 to thereby allow both the optical components and steering mechanisms of the medical tube 1030 to be utilized multiple times has several advantages over devices traditionally used to view inside the body, namely endoscopes. In particular, the assembly 1010 may be used multiple times without the need for time consuming and expensive procedures to sterilize the assembly. In contrast, an endoscope must be properly prepared and sterilized prior to reuse in order to reduce the risk that an infection could be transmitted from one patient to another. Furthermore, the patient need not be subjected to additional procedures in order to confirm that the medical tube 1030 has not been displaced. Medical personnel need only reconnect the coupling adaptor 1022 to the control unit 1050 in order to confirm that the tubing is in its proper place and, if not, re-advance the tubing to the position where it should be located.

An additional aspect of the optical medical tube and control unit assembly 1010 that can be seen in FIGs. 1 and 2 is a tube mounting unit 1042. The tube mounting unit 1042 may be used to couple tubing to the control unit 1050 and provide the ability of medical personnel to control flushing, aspiration and/or insufflation during use of the assembly 1010, which is described in more detail below. To give a brief example, the tube mounting unit 1042 may have one or more retention members 1044 for selectively holding tubing segments in place and one or more occlusion members 1048 for selectively controlling the flow of fluid from an external source through a lumen in the medical tube 1030. (It will be appreciated that the medical tube 1030 may have more than one lumen). Control of the retention members 1044 may be provided by one or more retention buttons 1036 or other retention control members to selectively allow placement or removal of a tubing segment from a retention location 1060, such as a channel, void, space, etc., in the tubing mounting unit 1042.

Likewise, buttons or flow control members 1038, which may be disposed along a handle portion 1054 of the control unit 1050 may enable control of flow through the tubing segments (not shown in FIGs. 1 and 2) disposed in the channel(s) 1060. This may be done, for example, by being biased into a position wherein an occlusion member 1048 pinches closed a tubing segment. Applying a force to a button 1038, which may be operatively connected to the tube mounting unit 1042 and an occlusion member 1048, may move the occlusion member from a first, closed position to a second, open position, wherein it no longer applies sufficient force to the tubing segment to pinch the tubing segment closed. Releasing force on the button 1038 may allow the occlusion member 1048 to return under bias to the first, closed position. By selectively pushing buttons 1038, medical personnel are able to control fluid flow into a lumen in the medical tube 1030 and selectively control which fluids are allowed to pass through the tubing segments into the medical tube (or the creation of a vacuum within the medical tube due to a vacuum in a connected tubing segment). (It will be appreciated that the function of buttons 1036 and 1038 could be combined to provide a single control button which enables mounting and removal of the tubing, along with selective occluding of flow through the tubing.)

Tubing mounting unit 1042 may comprise a plurality of locations for engaging a plurality of tubing sections to thereby provide for multiple functionalities, such as flushing of the optical fiber at the distal end of the medical tube 1030, aspiration of a fluid from the body of the patient, etc. These locations may be channels 1060 formed in the tube mounting unit 1042 or the interaction of the retention members 1044 and the body of the tube mounting unit 1042 to adequately secure the tubing segments.

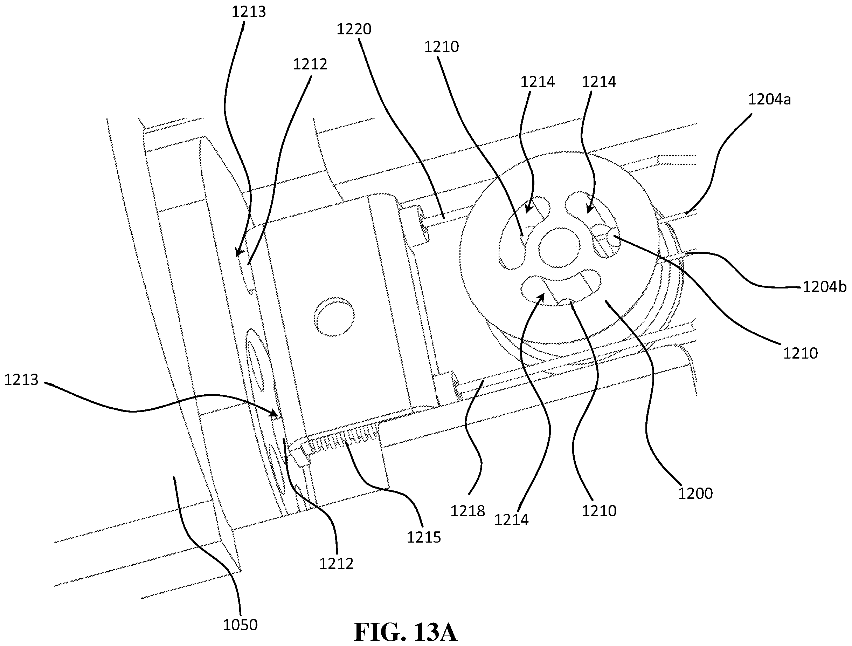

The optical medical tube and control unit assembly 1010 may also include a tube adaptor 1020 for operatively connecting the medical tube 1030 to a separate tubing section (see e.g. FIG. 13A) designed to be received by the tube mounting unit 1042. The tube adaptor 1020 may include a first section 1014 in fluid communication with a lumen of the medical tube 1030 configured to deliver medicine, nutrients and/or other solutions to a patient after placement of the medical tube 1030, or configured to provide aspiration and insufflation during or after placement of the medical tube 1030. The tube adaptor 1020 may also include a second section 1018 in fluid communication with a lumen of the medical tube 1030 configured to provide a way to flush the imaging device 1034 at the distal end of the medical tube 1030 to enable better image capture by the assembly 1010.

As was mentioned previously, the control unit 1050 may include an image capturing device and/or light source to enable viewing through the imaging device 1034. Thus, the housing 1046 may include a video screen (not shown) or a connector for attaching the housing to a viewing screen such as a monitor, a smart phone or other visual display to enable a physician or other medical personnel to view the tissue adjacent the distal end of the imaging device.

It will be appreciated that, although the discussion may be focused on optical tubes intended for use in the field of medicine, and more specifically optically guided feeding tubes, optical tube systems may have many uses in situations when there is a need to inspect an area that is inaccessible by other means. For example, optical tube systems are used to inspect large engines or turbines, have forensic applications in law enforcement, can be used in gunsmithing for inspecting the interior bore of a firearm, and are used by bomb disposal personnel to examine improvised explosive devices. Optical tube systems have even found use in architectural work as a way to visualize scale models of proposed buildings.

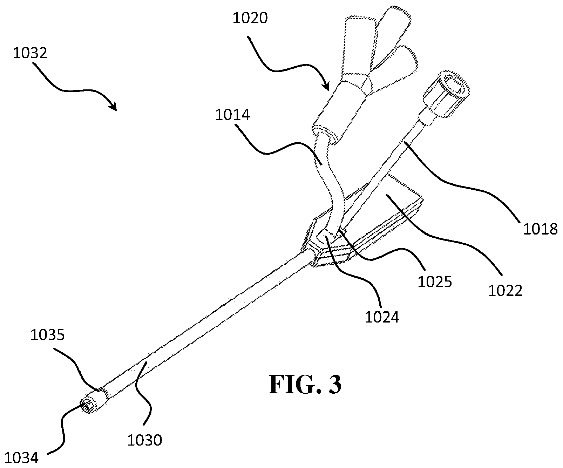

Turning now to FIG 3, there is shown a fragmented perspective view of an optical medical tube, generally indicated at 1030 and coupling adaptor 1022 forming an optical medical tube assembly, generally indicated at 1032. Optical medical tube assembly 1032 may include a medical tube 1030 having multiple lumens, including lumens for an imaging device 1034. The imaging device may comprise an optical fiber with a light ring or a camera on a wire including a light source. The optical medical tube assembly 1032 may also include a steering mechanism associated therewith. The tube 1030 may also include a lumen configured to deliver medication, nutrients and/or other solutions post-placement of tube 1030, lumens designed to flush the imaging device 1034, and steering members (not shown in FIG. 3).

In addition, optical medical tube 1030 may include a tube tip 1035 disposed so as to align the imaging device 1034 generally parallel with the center axis of tube 1030 which may facilitate transmission of a view at the distal end of the tip 1035 to the user of the assembly 1010. The tube tip 1035 may also help direct flushing of the imaging device 1034 and may provide an anchor point for the steering mechanism.

The medical tube 1030 may be coupled to a coupling adaptor 1022, which may be configured to be removably connected to a control unit 1050 of the assembly 1010. Coupling the tube 1030 to a coupling adaptor 1022 that is small and lightweight may be advantageous in that the coupling adaptor 1022 may go substantially unnoticed to the patient in which the tube 1030 has been placed increasing patient comfort while allowing the control unit 1050 to be quickly and easily reconnected to and/ or disconnected from the tube 1030 at a later time.

The optical medical tube assembly 1032 may also include a tube adaptor 1020. The tube adaptor 1020 may include a connector 1014 for connecting to devices configured for administering medicine, nutrients or other solutions after the tube 1030 has been placed. As an example, the connector 1014 may connect to devices configured for delivering a fluid for flushing or insufflation and also to devices configured for providing suction capabilities for aspirating a fluid from the body. Additionally, the tube adaptor 1020 may include a flush port 1018 for flushing the imaging device 1034. The flush port 1018 may be capped after placement of tubing 1030 unless reconfirmation that the tube 1030 is in its proper place is later needed.

Both the tube adaptor 1020 and the flush port 1018 may connect to the tube 1030 via the coupling adaptor 1022 in a manner that eliminates cross communication between lumens associated with the optical medical tube 1030. The coupling adaptor 1022 may have one or more channels therein configured so as to communicate with a specific lumen in the tube 1030. For example, the connector 1014 may be attached to a coupling adaptor 1022 at one end 1024 of a channel in the coupling adaptor 1022 so that if a fluid is introduced into connector 1014 it passes through the channel and only into a lumen configured for administration of medication, nutrients and/or other solutions. A second channel may exist in the coupling adaptor 1022 at the location where the flush port 1018 connects to the coupling adaptor 1022, such that the second channel only communicates with a lumen configured for flushing of the imaging device 1034.

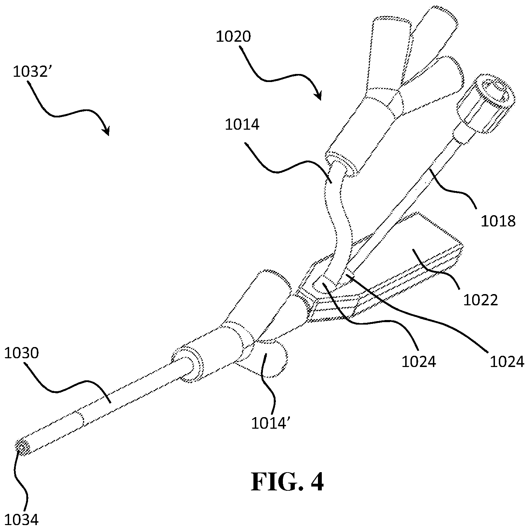

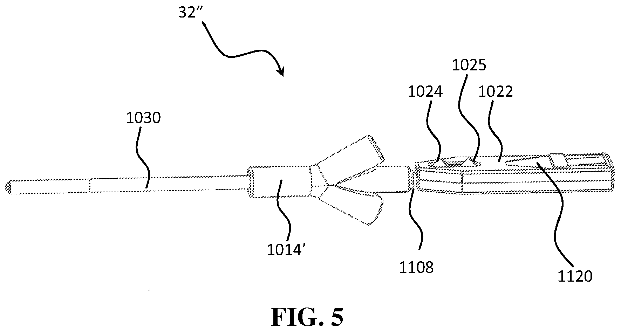

Turning to FIGs. 4 and 5, there are shown perspective views of additional aspects of optical medical tube assemblies according to principles of the present invention, generally indicated at 1032' and 1032" respectively. The assemblies 1032' and 1032" may include a second connector 1014'. Connector 1014' may be disconnected from coupling adaptor 1022 post-placement of a tube 1030 and be used to connect to devices configured for administering medication, nutrients and/or other fluids to a patient. Assemblies 1032' and 1032" may also include connector 1014 (not shown in FIG. 5) for connecting to a tubing section loaded on the tube mounting unit shown in FIG. 1 to provide the user of an assembly 1010 various functionalities during placement of the tube 1030.

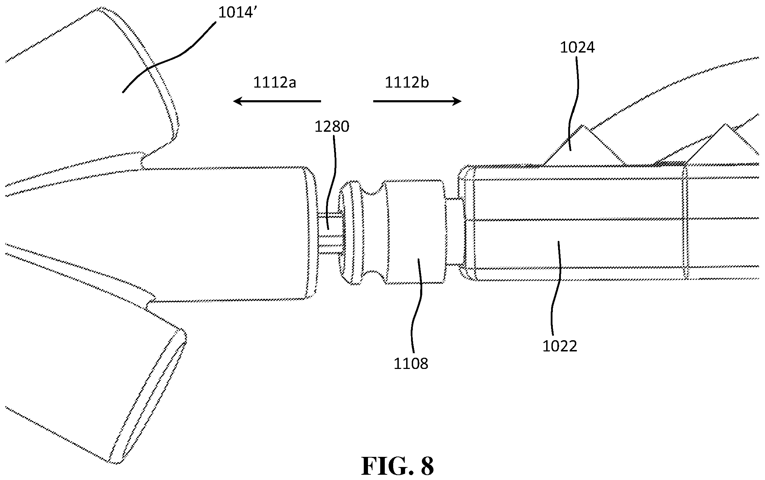

The coupling adaptor 1022 may be detached from the tube 1030 after placement at a connection point 1108 (FIG. 5) between the connector 1014' and the coupling adaptor 1022, as can be seen better in FIG. 8. It will be appreciated that there are various methods for connecting connector 1014' to coupling adaptor 1022 including use of a twist lock, luer lock, bayonet lock, barbed fitting, O-ring fittings, and the like. When the tube 1030 is intended to be left in a patient for an extended period of time, detachment of the coupling adaptor 1022 may be desirable so that the patient is as comfortable as possible. If confirmation that the tube is in its proper place is later needed, it is simple to attach a new coupling adaptor 1022 to the tube 1030 in order to have the ability to view and steer the tube 1030 if necessary.

An additional aspect that can be seen in FIG. 5 is the coupling adaptor 1022 having an optical fiber connector 1120. The optical fiber connector 1120 may have a snap-fit connection to the coupling adaptor 1022 and provide for removal of the optical fiber from the tube 1030. Thus allowing for reuse of the optical fiber following simple cleaning, such as swabbing the optical fiber with an alcohol swab. The ability to remove and reuse the optical fiber may be advantageous because typically the optical fiber which needs to be used with an optical medical tube assembly 1032, 1032', or 1032" can be expensive. For example, the optical fiber may need to have a sufficiently small enough outer diameter to allow a tube to be comfortably advanced through a narrow passageway while maintaining enough space in the tube for additional lumens and structures for various functionalities. Such optical fibers usually have to be made from more expensive materials because image quality from optical fibers made of cheaper materials is too poor for the intended purposes of the present invention. Thus, the ability to remove and reuse an optical fiber may be important to make the optical feeding tubes of the present invention cost effective by allowing less expensive, larger optical fibers to be used.

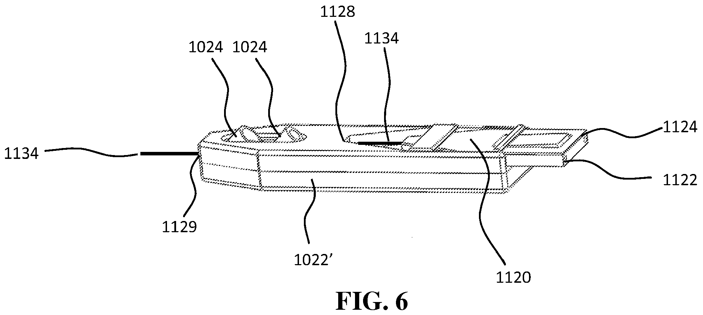

An example of how an optical fiber 1134 is removed from or attached to a coupling adaptor 1022 can be better seen in FIG. 6. The optical fiber 1134 may be removably attached to the connector 1120. The optical fiber 1134 may include a one or more fibers for transmitting light down a medical tube, and one or more fibers associated with an image capture device.

The connector 1120 have a releasable connection (e.g. a snap fit connector) with coupling adaptor 1022 in a manner that provides for the optical fiber 1134 aligning with both a light source at position 1124 and an image capturing device at position 1122. The optical fiber 1134 may be directed to a lumen in a tube 1030 (not shown) via a channel in coupling adaptor 122 that may run from an insertion point 1128 to an exit point 1129. When a tube 1030 is coupled to the coupling adaptor 1022, the optical fiber 1134 may be aligned to enter a lumen in the tube 1030 (or a stylet, which is describe in more detail below) when exiting the coupling adaptor 1022 at exit point 1129. By disconnecting the connector 1120 from adaptor 1022', the optical fiber 1134 can be slid out of the adaptor after use and then sterilized or cleaned for reuse when needed. Even if the optical fiber 1134 is sterilized prior to use on another patient, resterilizing the optical fiber is less expensive and time consuming that resterilizing an endoscope.

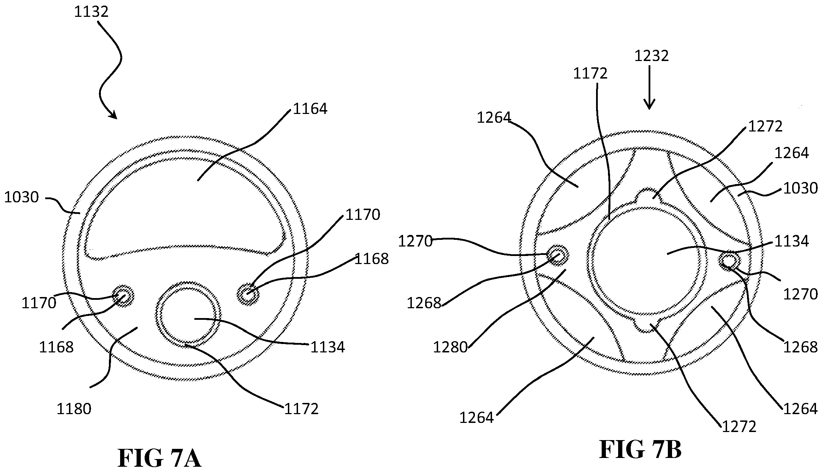

Turning to FIG. 7A, there is shown an end view of a medical tube 1030 of an optical medical tube assembly, generally indicated at 1132, made according to principles of the present invention. The tube 1030 includes a lumen 1164 that may be used for flushing, insufflations, or aspiration during or after placement of the tubing, or may be used for delivering medication, nutrients and/or other fluids to a patient post-placement. The tube 1030 may include a lumen for an imaging device 1134, such as an optical fiber. Substantially adjacent or surrounding the imaging device 1134 may be a lumen 1172 connected to the flush port 1018 (see e.g. FIG. 3). The lumen 1172 may be used to flush the imaging device to provide the user of an assembly 1010 with higher quality images for placing the tube 1030 or for confirming that tube 1030 has not been inadvertently displaced after it has been inside the patient over a period of time. As can be seen in FIG. 7A, the tube 1030 may be configured to substantially prevent cross communication between lumens 1172 and 1164.

The tube 1030 may also include multiple lumens 1170 for receiving steering members 1168, such as steering wires. Use of multiple steering wires 1168 may provide for multidirectional deflection of the optical medical tube 1132 during placement. One skilled in the art will appreciate that steering wires 1168 may be strings, lines, cables and the like so long as what is used can adequately deflect the tube 1030 so that a user of assembly 1010 may follow the pathway of a structure under examination, with minimum deflection or friction force upon the surrounding tissue, and to survey targeted examination sites.

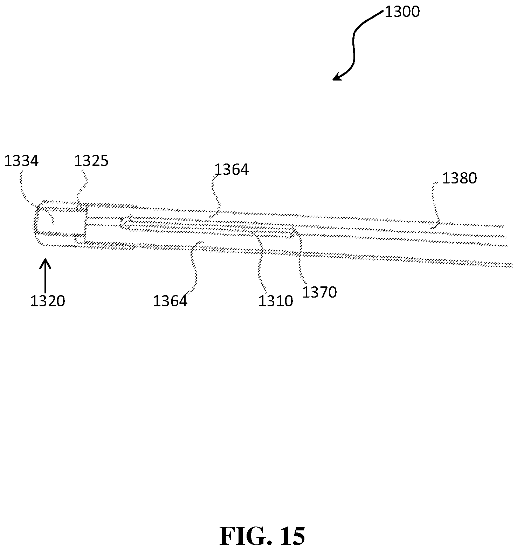

As is explained in further detail below, the optical medical tube assembly 1132 may include a tube tip (FIG. 15) to provide alignment for imaging at the distal end of the tube 1030. The tip may also aid in facilitating flow of fluids, nutrients and medication from lumens 1164 and 1172.

FIG. 7B, shows a front view of a tube 1030 and a stylet 1280 that may be used with an optical medical tube assembly, generally indicated at 1232, of the present invention. The stylet 1280 may include or, with the tube, define multiple lumens that perform substantially the same functions as described above. However, the generally X-shaped cross-section of the stylet 1280 may provide additional advantages. For example, the X-shaped configuration may allow the stylet 1280 to receive an imaging device such as optical fiber 1034, having a larger outer diameter than can be received by the lumen 1172 of the tube 1130 in FIG. 7A. This enables the use of less expensive optical fibers manufactured using, for example, plastic, which have a greater outer diameter (approximately 1.8 mm to 2.0mm) to provide comparable image quality to the more expensive smaller fibers.

The lumens 1264 in the medical tube 1230 may perform substantially the same functions as lumen 1164 in FIG. 7A. The lumens 1264 may be formed by the inner surface of the tube 1230 and various surfaces of the stylet. The stylet 1280 may also include lumens or channels 1272, which may be located substantially adjacent to the imaging device 1134 so as to provide for flushing of the imaging device when needed, and lumens 1270 for receiving steering wires 1268. As will be discussed in further detail below, the generally X-shaped cross-section of the stylet 1280 may provide the added advantage of increasing control of the movement of the tube 1230 at the distal end.

FIG. 8 shows a close-up perspective view of an optical medical tube similar to that shown in FIG. 5, with a connecting point or member 1108 disposed between the connector 1014' and the coupling adaptor 1022. This could be used, for example, with a stylet 1280 so that the coupling adaptor 1022 can be disconnected from the connector 1014' and the stylet 1280 can be pulled out of the tube 1030 when it is not needed. The remaining medical tube 1030 can be used for feeding, and administering medications, etc., and the stylet 1280 may be reinserted if desired for confirming tube placement, etc.

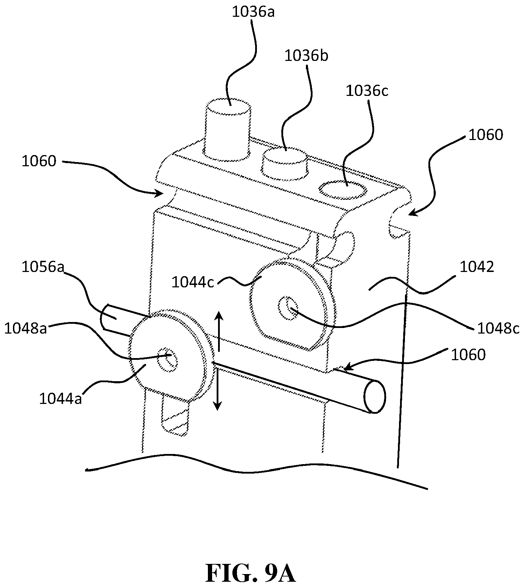

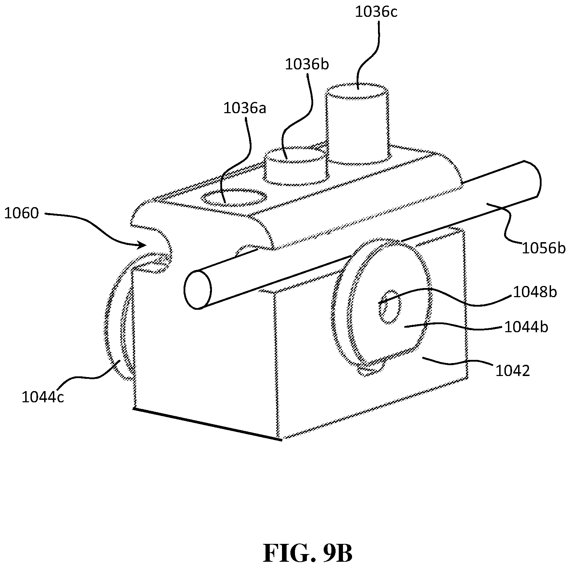

Turning now to FIGs. 9A and 9B, there are shown close-up, perspective views of a tube mounting unit 1042 which may form part of the control unit 1050. The tube mounting unit 1042 may comprise one or more retention locations 1060, such as channels, for engaging tubing sections according to principles of the present invention (such as a disposable tubing section described below). The tube mounting unit 1042 may also comprise one or more retention members, such as retention members 1044a - 1044c, located adjacent channels 1060 for holding tubing sections in channels 1060 during use. It will be appreciated that the one or more retention members 1044a - 1044c could be configured with a channel or other structure to hold the tubing segments without the need for channels 1060 in the tube mounting unit.

As shown in FIGs. 9A and 9B, the retention members 1044a - 1044c may be generally disc shaped and configured so that a portion of the retention member may be disposed alongside the tubing segment 1056a to hold the tubing segment in place. Thus, the retention members 1044a - 1044c may be selectively moveable between at least two positions, open or loading and closed or retaining, and can potentially be disposed in at least three or more positions.

According to one aspect of the invention, the retention members 1044a - 1044c are typically biased in a closed position by a biasing member, such as a spring, elastomeric material, etc. (not shown). In the embodiment shown in FIGs. 9A and 9B, the retention members 1044a - 1044c are connected to a biasing member disposed in the tube mounting unit 1042 by a shaft, bar, etc. 1048a-1048c. As will be explained below, the shaft, bar, etc. 1048a-1048c may also serve as an occlusion member 1048 for selectively terminating flow through a tubing segment 1056a disposed in one of the channels 1060 by, for example, pinching the tubing segment to selectively prevent flow therethrough.

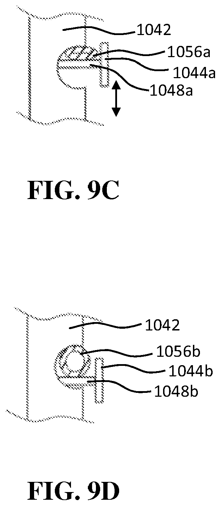

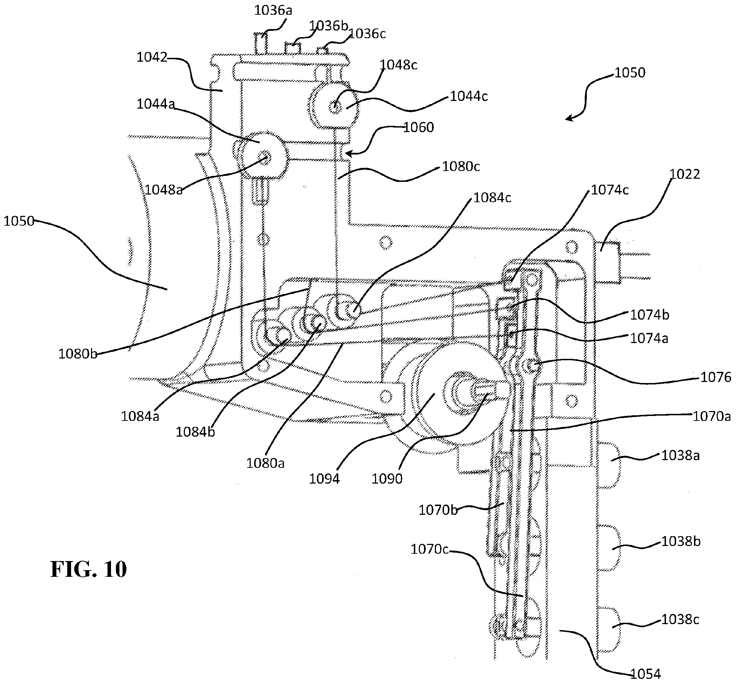

The retention member 1044a is shown biased into a first, closed position. (A close-up view is shown in FIG. 9C). The retention member 1044a is disposed alongside the channel 1060 to prevent the tubing segment 1056a from being withdrawn from the channel. As shown, the occlusion member 1048a is advanced upwardly to an extent where it will pinch closed the tubing segment and prevent fluid flow therethrough. Such a position would typically be associated with one of the buttons 1036a or 1038a (FIG. 10) being left undepressed. In this position, the retention member 1044a holds the tubing segment 1056a in place in the channel 1060 and the occlusion member 1048a substantially prevents flow therethrough. The retention member 1044a and the occlusion member 1048a may also be moved into additional positions as discussed below with respect to the other retention members 1044b, 1044c and occlusion members 1048b, 1048c.

The retention member 44b (FIGs. 9B and 9D) is in a second, closed position wherein the retention member 44b still prevents the tubing segment 1056b from being withdrawn from the channel 1060 in which it is disposed. Movement of the retention member 1044b into the second, closed position coincides with movement of the occlusion member 1048b downwardly from the first, closed or occluding position discussed above regarding occlusion member 48a, in to a second, open position wherein the occlusion member 1048b is no longer pinching closed the tubing segment 1056b. Thus, moving the retention member 1044b and occlusion member 1048b into the second position allows fluid flow through the tubing without releasing the tubing segment 1056b from the tube retention unit 1042. This may be accomplished by pressing one of the buttons, such as button 1036b or 1038b (FIG. 10) to move the associated retention member 1044b and occlusion member 1048b downwardly.

The retention member 1044c in FIG. 9A has been moved into a third, open position. This also results in the occlusion member 1048c being moved into a third, open position. With the retention member 1044c in the third, open position, the retention member no longer prevents the tubing segment (not show) from being removed from the channel 1060. As will be discussed in additional detail below, it is preferred that movement of the retention member 1044c into the third, open position require some action different than what would be required to move the retention member into the second, closed position. This may require, for example using two buttons (i.e. both buttons 1038c and 1036c), or require use of button 1036c where a user usually uses button 038c to move the retention member 1044c between the first, closed position and the second, closed position, thereby moving the occlusion member 1048c between the first, closed position and the second, open position. The movement into the third, open position may also be implemented by requiring the buttons 1036 and/or 1038 to be moved substantially beyond the normal range needed for movement of the retention members 1044 and occlusion members 1048 between the first and second positions, or by require a release which allows the button to be moved into a certain position. Thus, a user will not accidentally move the retention member 1044c into the third, open position and allow the tubing segment to be withdrawn by simply pressing too hard on the button used to open or close flow through the tubing.

Now discussing generally FIGs. 9A-10, in the first, closed position the occlusion members 1048a-1048c may be biased into position at or near the roof of channels 1060 so as to pinch closed tubing, which may be passing over the occlusion members 1048a-1048c, to thereby prevent flow of fluid in either direction through the tubing segment. Tube mounting unit 1042 may include plungers, buttons, knobs, or the like, such as 1036a - 1036c which may be used to overcome the force exerted by the biasing member to move the occlusion members 1048a - 1048c to a load position, such as is indicated by occlusion member 1044c in FIG. 9A, thus allowing access to channels 1060. (While such movement is discussed above as moving from a first, closed position to a third, open position, it will be appreciated that multiple positions may be provided, or that the retention members and occlusion members may be formed which operate independently of one another. Thus, any reference to first, second, etc., positions, is for convenience and shall not be deemed to be limiting).

In use, pushing down on plunger 1036c (FIG. 10) may provide sufficient force to move occlusion member 1044c to the load position as shown in FIG. 9A. After a tubing segment is positioned in the channel 1060, plunger 1036c may be released and a biasing element (such as a spring, band, etc., may cause the retention member 1044c to return to the first, closed position to thereby hold the tubing in channel 1060 and the occlusion member 1048a to pinch closed the tubing. The same may be repeated for some or all of the retention members and occlusion members so that a plurality of tubing segments are removeably attached to the tube mounting unit 1042 and selectively occluded.

Controlling flow of fluid through a tubing section located in channel 1060 may be accomplished by moving the occlusion members 1048a - 1048c between a plurality of positions using an actuation mechanism that may be operatively connected to the occlusion member 1044a - 1044c (such as the actuation mechanism of control unit 1050 shown in FIG. 10). For example, flow of a fluid through tubing section 1056a may be substantially prevented by occlusion member 1048a, which is in a closed position, whereas fluid may easily flow through tubing section 1056b because occlusion member 1048b is in an open position.

The actuation mechanism shown in FIG. 10 may allow the user of an optical medical tube and control unit assembly 1010 to easily control the amount of fluid passing through a tubing section held in a channel 1060 by moving the occlusion members 1048a - 1048c to a plurality of positions between, and including, the open and closed positions by applying force to, or removing the same force from, plungers, buttons, knobs, etc. 1038a - 1038c. As can be seen in FIG. 10, the control unit 1050 may include multiple buttons which can be used to perform a variety of functions while the assembly is in use, e.g. flushing, aspiration, and/or insufflation.

Buttons 1038a - 1038c may be located on handle 1054 to permit a user to grip and maneuver the assembly 1010 while simultaneously performing other various functions. Actuation of the occlusion members 1048a - 1048c to control flow through tubing may be accomplished using buttons 1038a - 1038c and the cable and lever actuation system, as shown in FIG. 10. For example, occlusion members 1048a - 1048c may be biased in a closed position (1048a in FIG. 9A, 9C) by including a biasing member, such as a spring, in the control unit 1050 that biases the occlusion members 1048a - 1048c towards the roof of the channels 1060. It is generally advantageous to have the occlusion members 1048a - 1048c biased in the closed position so that when the assembly 1010 is in use a fluid is not inadvertently introduced into or aspirated from the body. When a user of the assembly 1010 wishes to open a fluid pathway in a tubing segment that may be held in the channels 1060 of the tube mounting unit 1042, the user need only apply a force to one of the buttons or knobs 1038a - 1038c. For example, pushing button 1038a overcomes the force exerted by a biasing member associated with occlusion member 1048a when the force from pushing button 1038a is transferred to the occlusion member 1048a and applied in an opposite direction than the force applied by its associated biasing member. Overcoming the force exerted by the biasing member opens a fluid pathway in a section of tubing in communication with the occlusion member 1048a to allow fluid to pass therethrough (see tubing 1056b FIG. 9B, 9D).

In further detail, the button 1038a may be connected to lever 1070a at one end, which may be pivotably attached to the control unit 50 at pivot point 76. Cable 1080a, which attaches to occlusion member 1048a, may operatively connect the button 1038a to the occlusion member 1048a via its connection to the opposite end of the lever 1070a at an attachment point 1074a. (It will be appreciated that the cables 1080a - 1080c may alternatively be strings, wires, lines, strands, rods, bands, belts, straps, or the like) Depending on the orientation of the occlusion member 1048a on the control unit 1050 relative to the cable attachment point 1074a on the lever 1070a, cable 1080a may also have to be redirected using structures 1084a, which may be any structure that provides a fulcrum point for the cable 1080a to pivot around. As is shown in FIG. 10, because occlusion member 1048a is offset relative to the cable attachment point 1074a, structure 1084a is used as a fulcrum point so that the force generated by pushing button 38a is ultimately applied to the occlusion member 1048a in a direction substantially opposite the direction of force being exerted by a biasing member.

A fluid pathway may be opened in other channels 1060 of tube mounting connecter 1042 in similar fashion by actuation of the occlusion members 1048b or 1048c, or any number of occlusion members that may be associated with the tube mounting connecter 1042. It will be appreciated that the cable and lever system shown in FIG. 10 is intended to be exemplary of the actuation mechanisms that may be used in accordance with principles of the present invention, and that the optical tube and control unit assembly 1010 contemplated herein may utilize a variety of systems to control flow of fluid through tubing.

According to one aspect of the invention, the buttons 1038a - 1038c may be used to move occlusion members 1048a - 1048c from a closed, occluding position to an open, non-occluding position, but are unable in a normal use configuration to move the occlusion members sufficiently to move the retention members 1044a-1044c into an open or load position. Thus, buttons 1038a - 1038c may only be used to move the occlusion members 1048a - 1048c sufficiently to control flow through the tubing segments, but not to allow removal of the tubing segments from the channels. To load tubing segment to the tube mounting device 1042, retention members 1044a-1044c (and occlusion members 1044a - 1044c) must be moved using a force that is applied instead of or in addition to buttons 1038a - 1038c. Such separate force may be provided by pressing plungers 1036a - 1036c described above. Alternatively a force may be applied directly to retention members 1044a - 1044c, independent any separate structure. It will be appreciated that there are a variety of methods for applying a force to retention members 1044a - 1044c consistent with principles described herein.

By requiring a separate force to move the retention members 1044a - 1044c to the load position, the risk that tubing located in channel 1060 will accidently be displaced from the tube mounting unit 1042 during use of the assembly is reduced as retention members 1044a - 1044c block the exit of a piece of tubing from channel 1060 even when the occlusion members 1048a-1048c are in the open position. Therefore, a user of the assembly 1010 can be confident that he or she will be able to perform a desired function at the proper time by simply pressing buttons 1038a - 1038c.

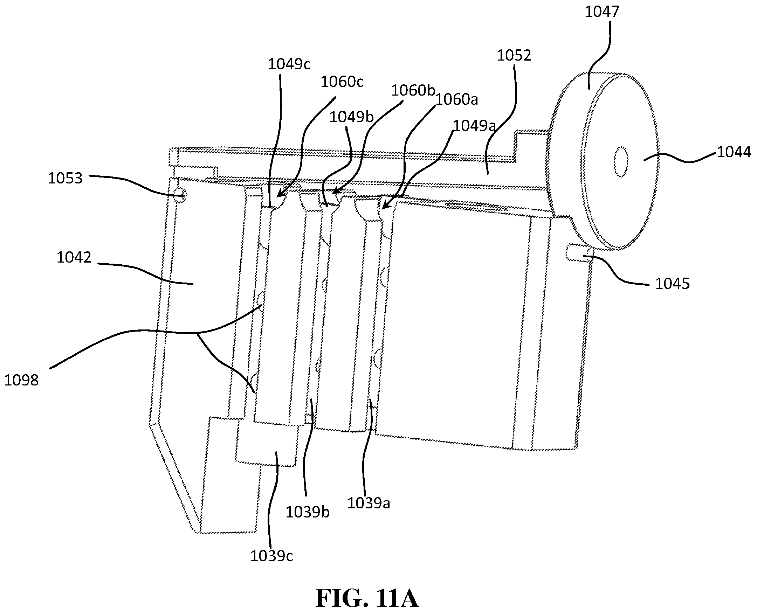

Turning now to FIGs. 11A - 11C, there is shown another tube mounting unit 1042, according to one aspect of the present invention. The tube mounting unit 1042 may comprise a body having one or more retention locations 1060, such as a space, void, channel etc. for receiving tubing sections, such as a disposable tubing section described in more detail below. The tube mounting unit 1042 may also comprise at least one retention member 1044 for holding tubing sections in the one or more retention locations 1060 of the body. The retention member 1044 may include an arm 1052 and a locking member 1045. The arm 1052 may be connected to the tube mounting unit 1042 such that the arm 1052 is located adjacent the retention locations 1060 when the retention member 1044 engages the locking member 1045. As shown in FIGs. 11A - 11C the arm 1052 may be pivotably connected to the tube mounting unit 1042 at location 1053. However, it will be appreciated that the arm 1052 may connect with the tube mounting unit 1042 in other manners as well.

The tubing mounting unit 1042 may removably receive one or more tubing segments in the retention locations 1060a - 1060c. The arm 1052 may be pivoted away from the tube mounting unit 1042 to allow access to the retention locations 1060a - 1060c. After one or more tubing segments are position in the retention locations 1060a - 1060c, the arm 1050 may be pivoted such that the retention member 1044 is in a position to engage the locking member 1045 to securely hold the one or more tubing segments in the retention locations 1060a -1060c. As shown in FIGs. 11A - 11C, locking member 1045 may be a projection disposed on the tube mounting unit 1042 and the retention member 1044 may include a sidewall 1047. The retention member 1044 may releasably engage the locking member 1045 by, for example, moving the locking member 1045 through a notch or void 1041 in the sidewall 1047 and rotating the retention member 1044 such that the locking member 1045 comes into contact with an inner surface 1043 of the sidewall 1047. It will be appreciated that the retention member 1044 may releasably engage the tubing mounting unit 1042 in a number of ways such that one or more tubing sections may be securely held in the retention locations 1060a - 1060c.

Also shown in FIG. 11A are a plurality of actuation members 1039a-1039c which may be disposed in channels 1041a-1041c in the tubing section 1042. The actuation member 1039a-1039c may be disposed so as to intersect the retention locations 1060a-1060c to thereby engage and selectively prevent or otherwise control flow through tubing disposed in the retention locations. The actuation members 1039a-1039c may be, for example, plungers which are biased into a first, closed position wherein they extend into the retention locations 1060a-1060c, respectively, to engage and control fluid flow through tubing segments.

In order to improve the ability of the actuation members 1039a-1039c to pinch closed tubing passing through the retention locations 1060a-1060c, an upper end 1049 of the actuation members may be tapered to a ridge so as to minimize the surface area engaging the tubing. The ridge may be disposed generally perpendicular to the length of the tubing to improve the ability to close the tubing.

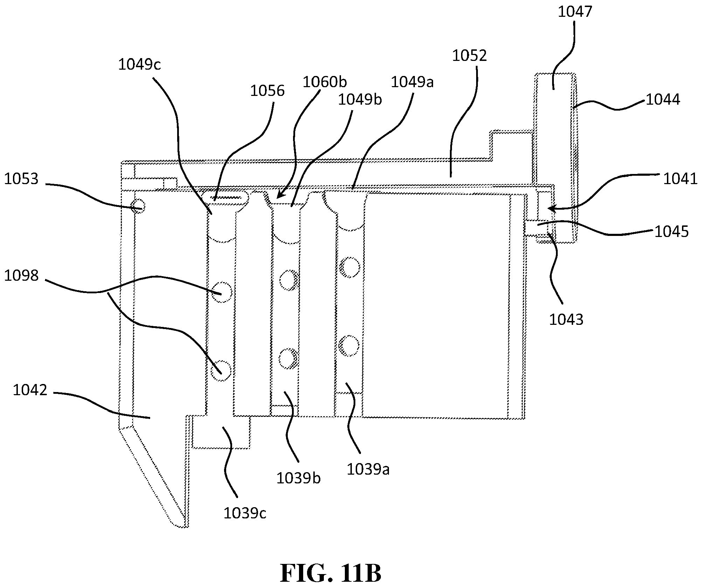

Referring now more particularly to FIGs. 11B - 11C, a tubing section 1056 is shown located in the retention location 1060c and disposed in communication with an end 1049c of the actuation member 1039c The actuation member 1039c may be biased towards the tubing segment 1056 (thus pinching the tubing closed) by a biasing member unless acted on by some other force. Thus as shown in FIG. 11B, the tubing section 1056 is closed and will prevent or substantially prevent flow therethrough.

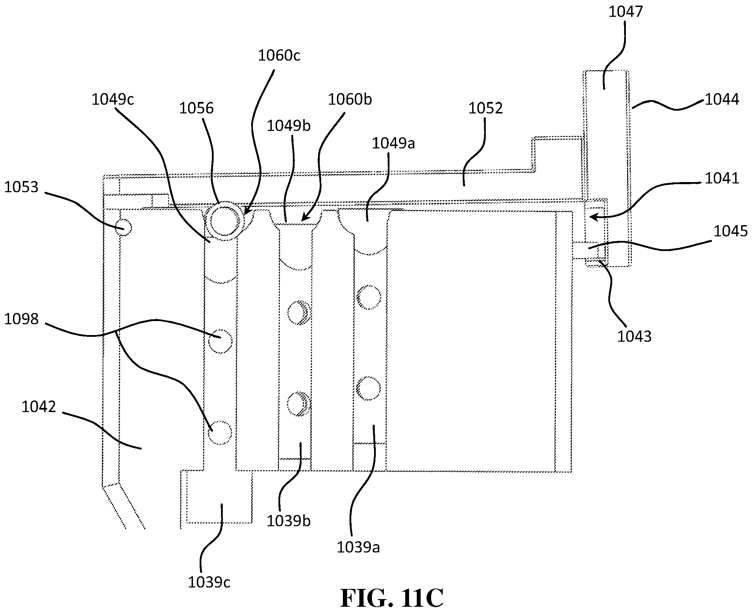

A cable and lever system, such as the system described in FIG. 10 above, may engage the actuation members 1039a-1039c at one or more locations 1098 via a number of mechanisms, such as set screws to pinch and hold the cable. The cable and lever system (or other system) may be used to apply a sufficient force to the actuation member 1039c to overcome the force being exerted by the biasing member to thereby move the actuation member 1039c away from the tubing 1056 and open a fluid flow channel through the tubing as shown in FIG. 11C. It will be appreciated that the actuation member 1039c may be movable between a plurality of positions between the open and closed position so that the actuation member may be used to partially close the tubing and thereby regulate rate of fluid flow through the tubing in addition to simply providing for flow and no flow configurations.

It will be appreciated that a fluid pathway may be opened in other tubing segments located in retention locations 1060a and 1060b in similar fashion, or any number of retention locations that may be associated with the tube mounting unit 1042.

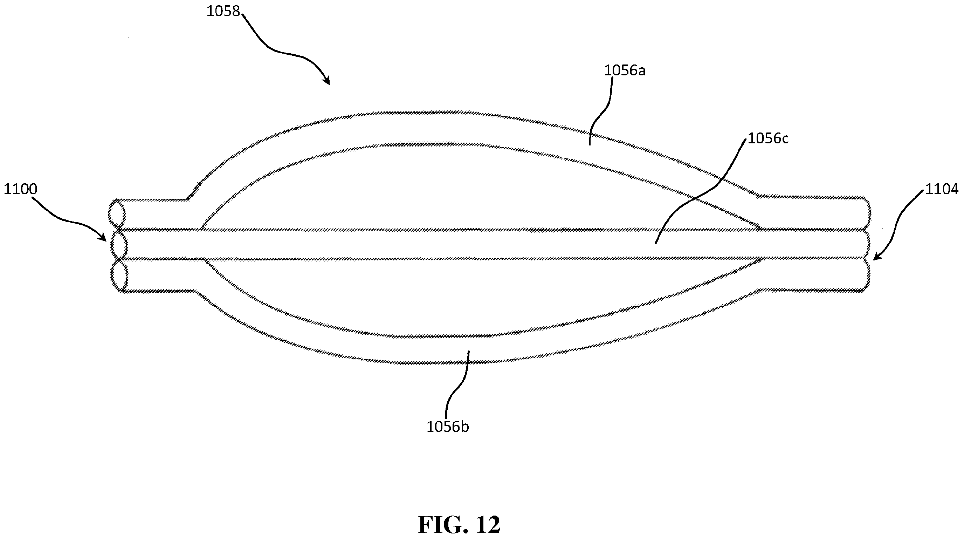

Turning now to FIG. 12, there is shown a side view of a tubing section, generally indicated at 1058, made in accordance with one aspect of the present invention. The tubing section 1058 may have multiple tube sections 1056a - 1056c each of which may be received by a retention location 1060, such as a channel (*See e.g.* FIG. 9A) or a space, void, etc. (*See e.g.* FIG. 11A) 1060 in the tube mounting unit 1042. The multiple tube segments 1056a - 1056c of the tubing section 1058 may be connected together at ends 1100 and 1104 and separated into individual tube sections between ends 1100 and 1104 to thereby facilitate mounting of the tubing section 1058 to tube mounting unit 1042.

End 1100 of the tubing section 1058 may be configured for quick and easy coupling to a tube adaptor (not shown) of the present invention. Additionally, the end 1104 may be configured for quick and easy coupling to devices designed to deliver fluid or air, or provide suction, to thereby operatively connect medical tubing 30 (FIG. 1) to such devices via the tubing section 1058. After use the tubing section may be discarded. Thus, unlike an endoscope which typically has lumens for flush, aspiration, and insufflation embedded in its handle making it a requirement that the endoscope go through a preparation and sterilization process prior to reuse, the control unit assembly 1050 may be reused almost immediately after it is removably disconnected from a medical tube 1030 because it does not come into contact with body fluids, etc. It will be appreciated that a single control unit 1050 may be used to place medical tubes 1030 in multiple patients, and/or reconfirm that a medical tube 1030 remains in its proper location in the body in multiple patients, over a short period of time.

It will also be appreciated that tubing section 1058 shown in FIG. 12 is only one example of a tubing section that may be used with the assembly 1010 according to principles of the present invention. Other examples of appropriate tubing sections that may be loaded on the tube mounting unit 1042 may include tubing sections having more or less tubes, or tubing sections whose tubes are not connected or only connected at one end. Also, the tubes associated with a tube adaptor 1020 may directly load on to the tube mounting unit 1042. These examples, though not exhaustive, illustrate the present inventions advantage over traditional endoscopes, namely that the tubing section and the control unit of the assembly can be readily disassociated from each other in order to allow the control unit 1050 to be removably connected to a different medical tube 1030 in a short amount of time and without the need for repeated sterilization.

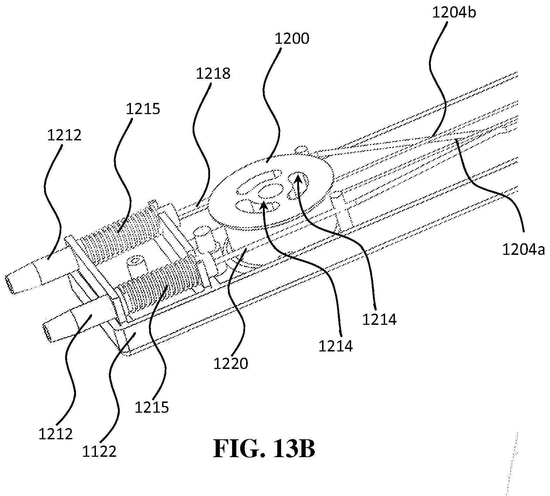

Now turning to FIG. 13A and 13B which show exemplary mechanisms for coupling the optics and steering mechanism of an optical medical tube with a control unit assembly according to principles of the present invention. As can be seen in FIG. 13, a steering mechanism having a pivotable member 1200, such as a wheel, may be easily and quickly coupled to a control unit 1050 of an optical tube and control unit assembly 1010 of the present invention. The steering mechanism may be incorporated in the coupling adaptor 1022 and may be made very small, e.g. about 0.5 inches in diameter, so that it provides little weight and bulk at the end of the medical tube 1030.

The steering mechanism may facilitate navigation of a tube 1030 (see FIG. 1) through a tortuous pathway. The steering mechanism may include multiple steering wires 1204a and 1204b that are wrapped around the wheel 1200. (Although not shown, it should be apparent from the above discussion that the steering wires 1204a and 1204b are associated with an optical medical tube of the present invention.) Steering wire 1204a may wrap around the wheel 1200 in a counterclockwise direction and steering wire 1204b may wrap around the wheel 1200 in a clockwise direction wire 1204a so as to provide for multidirectional movement of an optical medical tube of the present invention. For example rotation of the wheel 1200 in a clockwise fashion will result in steering wire 1204b being pulled towards the control unit 1050 and provide additional slack to steering wire 1204a, thus ultimately resulting in an associated optical medical tube being deflected in the direction of steering wire 1204b. It will be appreciated that the associated optical medical tube may be deflected in the opposite direction by rotating the wheel in a counterclockwise fashion. It will also be appreciated that more or less wires may be included in an optical tube and control unit assembly of the present invention, which in turn may provide a user of such an assembly with more or less control over the movement of an optical medical tube.

One advantage of the engagement of the steering wires 1204a and 1204b and the wheel 1200 is that the wires can be controlled in a very compact space. This allows the wheel and wires to be kept with the medical tube if desired.

The wheel 1200 may be coupled to the control unit 1050 via one or more slots 1214 in wheel 1200 and one or more pins 1210 located on the control unit 1050. (Of course, the configuration could also be reversed with the pin(s) being located on the wheel 1200 and the slot(s) being on the control unit 1050). Such a configuration for coupling the wheel 1200 to the control unit 50 provides for quick and easy connecting of the steering wires 1204a and 1204b so that a user may control movement of an optical medical tube via the control unit 1050.

It will be appreciated that other steering mechanisms may be used, such as, for example, a bar which pivots and has the steering wires 1204a and 1204b attached to opposing ends. The wheel configuration is believed to be advantageous as the wheel can be made to undergo multiple rotations if necessary, thus allowing the wheel to be very small in relative size.

Referring back to FIG. 10, a control unit 1050 may include a gear system 1094 which is operatively connected to the pins 1210 in FIG. 13A. Gear system 1094 may include a drive 1090 which, when rotated, actuates gear system 1094 thereby moving pins 1210 and turning wheel 1200 in a clockwise or counterclockwise direction depending on which way drive 1090 is rotated. Drive 1090 may be configured to receive a drive member, such as thumbwheel 1026 (FIG. 1) so that a user of assembly 1010 may easily rotate drive 1090 to steer an optical medical 1030 tube of the present invention. It will be appreciated that a steering mechanism comprised of a gear system 1094 is exemplary of the steering mechanisms of the present invention and that alternative steering mechanisms may be contemplated.

Fig. 13A also shows mounting structures 1212, such as ferules, associated with coupling adaptor 1022 which may be inserted into openings 1213 on control unit 1050. The mounting structures 1212 surround the ends of the optical wires 1218, 1220 and provide support thereto as the optical wires 1218, 1220 are brought into contact with the optical system (e.g. image capturing device and/or light source) disposed in the control unit 1050.

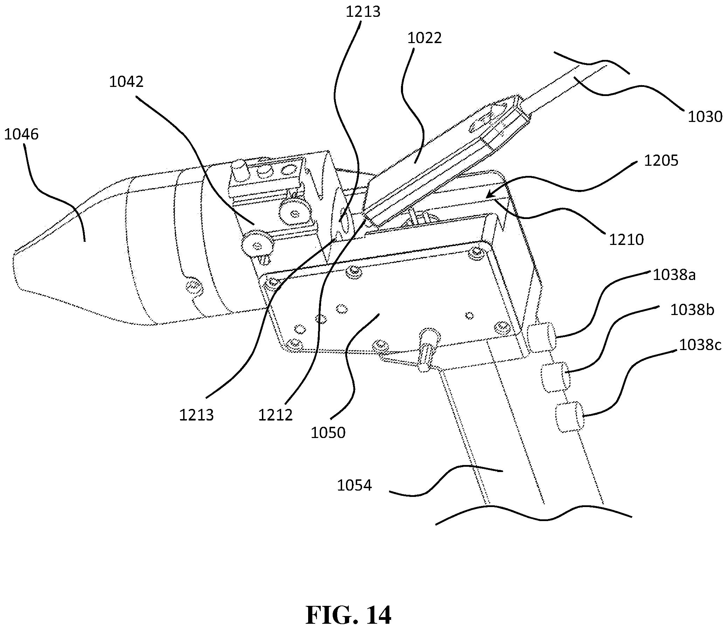

The mounting structures 1212 may include or be disposed in communication with biasing members 1215, such as a springs, elastomeric material, etc., which may facilitate alignment and seating of each mounting structure 1212 to a specified depth for substantially optimum interaction of the imaging device 134 (not shown) located in tube 1030 with an image capturing device and/or light source located in the control unit 1050. Alignment and seating of the posts 1212 to the specified depth within openings 1213 may provide for quick, high quality visual data while reducing the need for manual focusing and alignment with elements in the housing 1046 (FIG. 14).

It will be appreciated that the coupling adaptor 1022 may be removed from the control unit 1050 after initial placement of the feeding tube 1030. In order to allow a physician or other medical personnel to quickly verify that the distal end of the feeding tube 1030 is in the proper location, it is advantageous that the coupling adapter 1022 quickly and easily nest in the control unit. This may be accomplished by sliding the mounting structures 1212 into the openings 1213 and the control unit and pushing the coupling adapter 1022 down so that the projections 1210 engage the openings 1214 of the wheel 1200.

Turning now to FIG. 14, there is shown a fragmented, perspective view of a coupling adaptor 1022 being removably connected to a control unit 1050 according to principles of the present invention. Coupling adaptor 1022 may comprise a wheel 1200 of the steering mechanism described above positioned so as to couple with a steering engagement member, such as pins 1210 on control unit 1050 when coupling adaptor 1022 is seated in a recess 1205. As discussed above the coupling adaptor 1022 may include mounting structures 1212, such as posts of ferules, having a biasing member (not shown) which are intended to be insert into opening 1213 on control unit 1050 and which may facilitate alignment and seating of the biased mounting structure 1212 (and the end of the optic wires 1204a and 1204b to a specific depth. Additionally, openings 1213 may be chamfered in order to further facilitate alignment of the various components of an optical medical tube 1030 and the control unit 1050 when coupling adaptor 1022 is seated in recess 1205. The control unit 1050 can be used to control visualization, flow of fluids into or out of the tube 1030, and steering when the coupling adaptor 1022 is attached, and then be removed whenever convenient.

FIG. 15 shows a perspective, cutaway view of a stylet 1380 and tube tip 1320 at the distal end of a medical tube, generally indicated at 1300. The stylet 1380 is generally similar to the stylet 1280 referred to in FIG. 7B. One aspect of the present invention that is more readily seen in FIG. 15 is the tube tip 1320. The tube tip 1320 is attached to the tube 1300 so that there is a standoff or gap 1325 between the tip 1320 and the distal end of the stylet 1380. The gap 1325 may facilitate flushing, aspiration, and/or insufflation by ensuring that sufficient space exists between the tube tip 1320 and one or more lumens in the stylet 1380. Likewise, the gap 1325 may facilitate flow of medication or nutrients from lumens 1334 past the tube tip 1320.

Also shown in FIG. 15 is a cutaway 1364 in the stylet 1380. The cutaway 1364 is formed by removing a portion of stylet 1380 at the distal end of the tube 1300. It will be appreciated that the stylet 1380 may include one or more cutaways 1364 and that the one or more cutaways 1364 may provide increased deflection at the distal tip of the tube 1300, thus allowing the tube 1300 to be more easily advanced through complex and tortuous pathways. The generally X-shaped extrusion profile of the stylet 1380 in conjunction with one or more cutaways 1364 may allow for tubes with steering capabilities that are less expensive to manufacture than tubes made with multiple durometers of materials attached end to end to obtain similar steering capabilities.

Additionally, steering member (not shown) may be disposed in communication with the stylet For example, steering wires may pass through the lumen 1370 and attach at tube tip 1320, or at some other location near the distal end of the medical tube 1300. Thus, pulling a steering wire in a direction away from the distal end of the medical tube will deflect the distal end away from the center axis of the tube 1300. One skilled in the art will appreciate that the cutaway 1364 may allow the distal end to be deflected upon less force being used to pull a steering wire and/or exaggerate the amount the distal end of the tube 1300 is deflected.

There may be many other ways to implement the invention. Various functions and elements described herein may be partitioned differently from those shown without departing from the scope of the claims. Various modifications to these embodiments will be readily apparent to those skilled in the art, and generic principles defined herein may be applied to other embodiments. Thus, many changes and modifications may be made to the invention, by one having ordinary skill in the art, without departing from the scope of the claims. Additionally, all structural and functional equivalents to the elements of the various embodiments described throughout this disclosure that are known or later come to be known to those of ordinary skill in the art are expressly incorporated herein by reference and intended to be encompassed by the invention. Moreover, nothing disclosed herein is intended to be dedicated to the public regardless of whether such disclosure is explicitly recited in the above description.

There is thus disclosed an improved optically guided medical tube and control unit assembly and methods of using the same that reduces risks associated with the placement of medical tubes inside the body of a patient and may be used to reconfirm proper placement after the medical tube has been associated with the patient for an extended period of time.

## Claims

1. A feeding tube (1030, 1300) comprising:
an elongate body forming a tube and having a distal end and a proximal end;
an imaging device (1034, 1134) for viewing an area adjacent the distal end of the elongate body;
a stylet (1280, 1380) for receiving the imaging device, wherein the stylet is removably disposed in the elongate body; wherein lumens are formed by the inner surface of the tube and various surfaces of the stylet, said lumens being configured to deliver medication and/or nutrients to a patient in whom said feeding tube has been placed; **characterized in that** the stylet has an X-shaped cross-section and wherein
a tube tip (1035, 1320) is attached to the elongate body such that there is a gap between the tube tip and a distal end of the stylet, wherein the gap (1325) is configured to facilitate said flow of medication and/or nutrients past the tube tip.

2. The feeding tube according to claim 1, wherein the imaging device is removably received by the stylet.

3. The feeding tube according to claim 1, wherein the imaging device comprises an optical fiber (1034).

4. The feeding tube according to claim 3, wherein the X-shaped cross-section has a central lumen (1172) which allows the stylet to receive an optical fiber having an outer diameter of between about 1.8 mm and 2.0 mm.

5. The feeding tube according to claim 1, wherein the elongate body has an inner surface and the stylet has an outer surface, and wherein the inner surface of the elongate body and the outer surface of the stylet form at least one lumen (1264).

6. The feeding tube according to claim 1, further comprising a steering member (1268) disposed in communication with the stylet for deflecting the stylet to thereby deflect the distal end of the elongate body.

7. The feeding tube according to claim 6, wherein the stylet comprises at least one cutaway section to facilitate deflection of the distal end of the elongate body.

8. The feeding tube according to claim 1, wherein the tube tip is disposed on the elongate body for aligning the imaging device generally parallel with the center axis of the elongate body.

9. The feeding tube according to claim 6, wherein the steering member comprises a wire (1168, 1204a, 1204b) which is operationally connectable to a control unit (1050) which may be used to steer the feeding tube.

10. A feeding tube according to claim 1 and a control unit (1050), wherein the feeding tube further comprises a coupling adaptor (1022) for removably connecting the feeding tube to the control unit.

11. The feeding tube and control unit according to claim 10, wherein the control unit further comprises a light source and wherein, when the feeding tube is coupled to the control unit, the imaging device may be aligned so as to allow light from a light source located in a housing (1046) in the control unit to be directed through the imaging device to a distal end of the feeding tube.

## Patentansprüche

1. Ernährungstubus (1030, 1300), aufweisend:
einen länglichen Körper, der einen Tubus bildet und der ein distales Ende und ein proximales Ende aufweist;
eine Bildgebungsvorrichtung (1034, 1134) zum Betrachten eines Gebietes benachbart dem distalen Ende des länglichen Körpers;
einen Mandrin (1280, 1380) zur Aufnahme der Bildgebungsvorrichtung, wobei der Mandrin in dem länglichen Körper entfernbar angeordnet ist; wobei durch die Innenfläche des Tubus und verschiedene Flächen des Mandrin Lumina gebildet werden, wobei die Lumina konfiguriert sind, um Arzneimittel und/oder Nahrung einem Patienten zuzuführen, in den dieser Ernährungstubus platziert wurde; **dadurch gekennzeichnet, dass** der Mandrin einen X-förmigen Querschnitt aufweist und wobei
eine Tubusspitze (1035, 1320) an dem länglichen Körper befestigt ist, derart, dass ein Spalt zwischen der Tubusspitze und dem distalen Ende des Mandrin vorhanden ist, wobei der Spalt (1325) konfiguriert ist, um das Vorbeifließen von Arzneimitteln und/oder Nahrung an der Tubusspitze zu erleichtern.

2. Ernährungstubus nach Anspruch 1, wobei die Bildgebungsvorrichtung durch den Mandrin entfernbar aufgenommen ist.

3. Ernährungstubus nach Anspruch 1, wobei die Bildgebungsvorrichtung eine Lichtleitfaser (1034) aufweist.

4. Ernährungstubus nach Anspruch 3, wobei der X-förmige Querschnitt ein zentrales Lumen (1172) aufweist, das ermöglicht, dass der Mandrin eine Lichtleitfaser aufnimmt, deren Außendurchmesser zwischen 1,8 mm und 2,0 mm beträgt.

5. Ernährungstubus nach Anspruch 1, wobei der längliche Körper eine Innenfläche aufweist und der Mandrin eine Außenfläche aufweist, und wobei die Innenfläche des länglichen Körpers und die Außenfläche des Mandrin mindestens ein Lumen (1264) bilden.

6. Ernährungstubus nach Anspruch 1, weiter aufweisend ein Lenkelement (1268), das in Kommunikation mit dem Mandrin angeordnet ist, und zwar für ein Auslenken des Mandrin, um dadurch das distale Ende des länglichen Körpers auszulenken.

7. Ernährungstubus nach Anspruch 6, wobei der Mandrin mindestens einen weggeschnittenen Abschnitt aufweist, um das Auslenken des distalen Endes des länglichen Körpers zu erleichtern.

8. Ernährungstubus nach Anspruch 1, wobei die Tubusspitze an dem länglichen Körper angeordnet ist, um die Bildgebungsvorrichtung im Wesentlichen parallel zur Mittelachse des länglichen Körpers auszurichten.

9. Ernährungstubus nach Anspruch 6, wobei das Lenkelement einen Draht (1168, 1204a, 1204b) aufweist, der mit einer Steuereinheit (1050) funktionsmäßig verbunden werden kann, die verwendet werden kann, um den Ernährungstubus zu lenken.

10. Ernährungstubus nach Anspruch 1 und Steuereinheit (1050), wobei der Ernährungstubus weiter einen Verbindungsadapter (1022) aufweist, um den Ernährungstubus mit der Steuereinheit lösbar zu verbinden.

11. Ernährungstubus und Steuereinheit nach Anspruch 10, wobei die Steuereinheit weiter eine Lichtquelle aufweist und wobei, wenn der Ernährungstubus mit der Steuereinheit verbunden ist, die Bildgebungsvorrichtung so ausgerichtet werden kann, dass ermöglicht wird, dass Licht von einer Lichtquelle, die sich in einem Gehäuse (1046) in der Steuereinheit befindet, durch die Bildgebungsvorrichtung hindurch zu einem distalen Ende des Ernährungstubus geleitet wird.

## Revendications

1. Tube d'alimentation (1030, 1300) comprenant :
un corps allongé formant un tube et ayant une extrémité distale et une extrémité proximale ;
un dispositif d'imagerie (1034, 1134) pour visualiser une zone adjacente à l'extrémité distale du corps allongé ;
un stylet (1280, 1380) pour recevoir le dispositif d'imagerie, dans lequel le stylet est disposé de manière amovible dans le corps allongé ; dans lequel des lumières sont formées par la surface intérieure du tube et diverses surfaces du stylet, lesdites lumières étant configurées pour délivrer un médicament et/ou des nutriments à un patient dans lequel ledit tube d'alimentation a été placé ;
**caractérisé en ce que** le stylet présente une section transversale en forme de X et dans lequel un embout de tube (1035, 1320) est fixé au corps allongé de telle sorte qu'il existe une fente entre l'embout de tube et une extrémité distale du stylet, dans lequel l'espace (1325) est configuré pour faciliter ledit écoulement de médicament et/ou de nutriments au-delà de l'embout de tube.

2. Tube d'alimentation selon la revendication 1, dans lequel le dispositif d'imagerie est reçu de manière amovible par le stylet.

3. Tube d'alimentation selon la revendication 1, dans lequel le dispositif d'imagerie comprend une fibre optique (1034).

4. Tube d'alimentation selon la revendication 3, dans lequel la section transversale en forme de X a une lumière centrale (1172) qui permet au stylet de recevoir une fibre optique ayant un diamètre extérieur compris entre environ 1,8 mm et 2 mm.

5. Tube d'alimentation selon la revendication 1, dans lequel le corps allongé a une surface interne et le stylet a une surface extérieure, et dans lequel la surface intérieure du corps allongé et la surface extérieure du stylet forment au moins une lumière (1264).

6. Tube d'alimentation selon la revendication 1, comprenant en outre un élément de direction (1268) disposé en communication avec le stylet pour dévier le stylet afin de dévier ainsi l'extrémité distale du corps allongé.

7. Tube d'alimentation selon la revendication 6, dans lequel le stylet comprend au moins un tronçon découpé pour faciliter la déviation de l'extrémité distale du corps allongé.

8. Tube d'alimentation selon la revendication 1, dans lequel l'embout de tube est disposé sur le corps allongé pour aligner le dispositif d'imagerie généralement parallèle à l'axe central du corps allongé.

9. Tube d'alimentation selon la revendication 6, dans lequel l'élément de direction comprend un fil (1168, 1204a, 1204b) qui peut être relié de manière opérationnelle à une unité de commande (1050) qui peut être utilisée pour diriger le tube d'alimentation.

10. Tube d'alimentation selon la revendication 1 et une unité de commande (1050), dans lequel le tube d'alimentation comprend en outre un adaptateur de couplage (1022) pour relier de manière amovible le tube d'alimentation à l'unité de commande.

11. Tube d'alimentation et unité de commande selon la revendication 10, dans lequel l'unité de commande comprend en outre une source de lumière et dans lequel, lorsque le tube d'alimentation est couplée à l'unité de commande, le dispositif d'imagerie peut être aligné de manière à permettre à de la lumière provenant d'une source de lumière située dans un boîtier (1046) à l'intérieur de l'unité de commande d'être dirigée à travers le dispositif d'imagerie vers une extrémité distale du tube d'alimentation.
